(19) Europäisches Patentamt
European Patent Office
Office européen des brevets

(11) **EP 4 067 393 A1**

(12) **EUROPEAN PATENT APPLICATION**
published in accordance with Art. 153(4) EPC

(43) Date of publication:
**05.10.2022 Bulletin 2022/40**

(21) Application number: **21787695.2**

(22) Date of filing: **13.04.2021**

(51) International Patent Classification (IPC):
$C08F\ 20/06$ (2006.01)  $C08K\ 5/00$ (2006.01)
$C08K\ 5/13$ (2006.01)  $C08K\ 5/109$ (2006.01)
$C08J\ 3/24$ (2006.01)  $A61L\ 15/60$ (2006.01)

(52) Cooperative Patent Classification (CPC):
**A61L 15/60; C08F 20/06; C08J 3/24; C08K 5/00; C08K 5/109; C08K 5/13**

(86) International application number:
**PCT/KR2021/004673**

(87) International publication number:
**WO 2021/210902 (21.10.2021 Gazette 2021/42)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **13.04.2020 KR 20200044874**

(71) Applicant: **Lg Chem, Ltd.**
**Seoul 07336 (KR)**

(72) Inventors:
• **YUN, Haesung**
  **Daejeon 34122 (KR)**
• **JUNG, Seonjung**
  **Daejeon 34122 (KR)**
• **LEE, Ji Seok**
  **Daejeon 34122 (KR)**
• **CHOI, Hyungsam**
  **Daejeon 34122 (KR)**
• **KIM, Byungguk**
  **Daejeon 34122 (KR)**
• **KIM, Sanggon**
  **Daejeon 34122 (KR)**

(74) Representative: **Goddar, Heinz J.**
**Boehmert & Boehmert**
**Anwaltspartnerschaft mbB**
**Pettenkoferstrasse 22**
**80336 München (DE)**

(54) **SUPER ABSORBENT POLYMER AND METHOD FOR PRODUCING SAME**

(57) According to this invention, superabsorbent polymer that may continuously and safely exhibit improved bacterial growth inhibition property and deodorization property, without deterioration of the properties of superabsorbent polymer such as centrifuge retention capacity and absorption under pressure, and the like, and a method for preparing the same are provided.

EP 4 067 393 A1

**Description**

**BACKGROUND OF THE INVENTION**

**(a) Field of the Invention**

Cross-reference to Related Application

This application claims the benefit of Korean Patent Application No. 10-2020-0044874 filed on April 13, 2020 and Korean Patent Application No. 10-2021-0047956 filed on April 13, 2021 with the Korean Intellectual Property Office, the disclosures of which are herein incorporated by reference in their entirety.

This invention relates to superabsorbent polymer that can continuously and safely exhibit improved bacterial growth inhibition property and deodorization property, without deterioration of the properties of superabsorbent polymer such as centrifuge retention capacity and absorption under pressure, and the like, and a method for preparing the same

**(b) Description of the Related Art**

Super absorbent polymer (SAP) is synthetic polymer material that can absorb moisture of 500 to 1000 times of self-weight, and is also named differently as super absorbency material (SAM), absorbent gel material (AGM), etc. according to developing companies. The superabsorbent polymer began to be commercialized as sanitary items, and currently, it is being widely used as hygienic goods such as disposable diapers for children, and the like, water-holding material for soil, water stop material for civil engineering and architecture, sheets for raising seedling, freshness preservatives in the field of food circulation, fomentation material, and electric insulators.

However, such superabsorbent polymer is being most widely applied for hygienic goods or disposable absorbent products such as diapers for children or diapers for adults. Among them, in case applied for diapers for adults, secondary odor due to bacterial growth causes significant discomfort to consumers. In order to solve the problem, previously, an attempt to incorporate various bacterial growth inhibition components, or deodorization or antibacterial components in superabsorbent polymer, and the like has been made.

However, when incorporating antibacterial agent for inhibiting bacterial growth in superabsorbent polymer, it was not easy to selectively incorporate antibacterial components that exhibit excellent bacterial growth inhibition property and deodorization property, and yet, are not harmful to the human body and meet economical efficiency, and do no deteriorate basic properties of superabsorbent polymer.

For example, there has been an attempt to incorporate an antibacterial component containing antibacterial metal ion such as silver, copper, and the like, for example, copper oxide, in superabsorbent polymer. Such antibacterial metal ion-containing component may destroy cell walls of microorganisms such as bacteria to kill bacteria having an enzyme that may induce odor in superabsorbent polymer, thereby endowing deodorization property. However, the metal ion-containing component is classified as BIOCIDE substance that may kill even microorganisms beneficial to the human body. Thus, in case the superabsorbent polymer is applied to hygienic goods such as diapers for children or adults, incorporation of the metal ion-containing antibacterial components is excluded as much as possible.

Meanwhile, previously, when incorporating antibacterial agents for inhibiting bacterial growth in superabsorbent polymer, a method of mixing a small amount of the antibacterial agent with superabsorbent polymer was mainly applied. However, in case such a mixing method is applied, it was difficult to uniformly maintain bacterial growth inhibition property as time passes. Moreover, such a mixing method may cause non-uniform coatability and delamination of the antibacterial components during the process of mixing superabsorbent polymer and antibacterial agent, and it was required to install new equipment for mixing.

Thus, there is a continued demand for the development of technology relating to superabsorbent polymer that can exhibit excellent bacterial growth inhibition property and deodorization property without deterioration of basic properties of superabsorbent polymer.

**SUMMARY OF THE INVENTION**

It is an object of the invention to provide superabsorbent polymer that can maintain excellent basic properties such as centrifuge retention capacity and absorption under pressure, and yet, continuously and safely exhibit improved bacterial growth inhibition property and deodorization property, and a method for preparing the same.

According to one embodiment of the invention, there is provided superabsorbent polymer comprising:

base resin comprising crosslinked polymer of acrylic acid-based monomers having acid groups, at least a part of the acid groups being neutralized, and an internal crosslinking agent,

wherein at least a part of the base resin is surface-treated by a first surface crosslinking agent, and the first surface crosslinking agent is one or more selected from the cationic compounds, terpene-based compounds, phenol-based compounds, and phosphoric acid-based compounds.

According to another embodiment of the invention, there is provided a method for preparing superabsorbent polymer, comprising steps of:

conducting crosslinking polymerization of acrylic acid-based monomers having acid groups, at least a part of the acid groups being neutralized, and an internal crosslinking agent, to form hydrogel polymer;
drying, grinding and classifying the hydrogel polymer to form base resin; and
crosslinking the surface of the base resin, in the presence of a first surface crosslinking agent,
wherein the first surface crosslinking agent is one or more selected from cationic compounds, terpene-based compounds, phenol-based compounds, and phosphoric acid-based compounds.

According to still another embodiment of the invention, there is provided an article comprising the superabsorbent polymer.

The superabsorbent polymer of this invention can selectively inhibit the growth of bacteria harmful to human body and inducing secondary odor, thus exhibiting excellent bacterial growth inhibition property and deodorization property.

And, since the superabsorbent polymer is surface crosslinked using a surface crosslinking agent of a specific structure having excellent antibacterial and deodorization performance, it can stably exhibit excellent bacterial growth inhibition property and deodorization property for a long time, and can maintain excellent centrifuge retention capacity and absorption under pressure without deterioration of properties due to the addition of other antibacterial agents.

Thus, the superabsorbent polymer can be very preferably applied for various hygienic goods such as diapers for adults, in which secondary odor particularly becomes a problem.

## DETAILED DESCRIPTION OF THE EMBODIMENTS

The terms used herein are only to explain specific embodiments, and are not intended to limit the present invention. A singular expression includes a plural expression thereof, unless it is expressly stated or obvious from the context that such is not intended. As used herein, the terms "comprise", "equipped" or "have", etc. are intended to designate the existence of practiced characteristic, number, step, constructional element or combinations thereof, and they are not intended to preclude the possibility of existence or addition of one or more other characteristics, numbers, steps, constructional elements or combinations thereof.

Meanwhile, throughout the specification, a C1 to 20 alkyl group may be a linear, branched or cyclic alkyl group. Specifically, a C1 to 20 alkyl group may be a C1 to 18 linear alkyl group; a C1 to 10 linear alkyl group; a C1 to 5 linear alkyl group; a C 3 to 20 branched or cyclic alkyl group; a C3 to 18 branched or cyclic alkyl group; or a C 3 to 10 branched or cyclic alkyl group. As specific examples, methyl, ethyl, n-propyl, iso-propyl, n-butyl, iso-butyl, tert-butyl, n-pentyl, isopentyl, neo-pentyl, or cyclohexyl group, and the like may be mentioned, but not limited thereto.

Although various modifications can be made to the invention and the present invention may have various forms, specific examples will be illustrated and explained in detail below. However, it should be understood that these are not intended to limit the invention to specific disclosure, and that the invention includes all the modifications, equivalents or replacements thereof without departing from the spirit and technical scope of the invention.

Hereinafter, a superabsorbent polymer and a method for preparing the same according to the embodiments of the invention will be explained in detail.

For reference, the term "polymer" or "resin" used herein means the polymerized state of acrylic acid-based monomers, and may include those of all moisture content ranges, all particle diameter ranges, all surface crosslinked states or processed states. Among the polymers, those having moisture content of about 40 wt% or more after polymerized and before dried may be designated as hydrogel polymer. And, among the polymers, those having particle diameters of 150 $\mu$m or less may be designated as "fines".

As used herein, the term "base resin" or "base resin powder" means particles or powders made by drying and grinding of polymer of acrylic acid-based monomers, and it means polymer that is not surface modified or surface crosslinked as explained later.

The superabsorbent polymer according to one embodiment comprises base resin comprising crosslinked polymer of acrylic acid based monomers having acid groups, at least a part of the acid groups being neutralized, and an internal crosslinking agent, wherein at least a part of the base resin is surface-treated by a first surface crosslinking agent, and the first surface crosslinking agent is one or more selected from the group consisting of cationic compounds, terpene-based compounds, phenol-based compounds, and phosphoric acid-based compounds.

Previously, in order to secure antibacterial and deodorization properties in superabsorbent polymer, metal compounds having antibacterial function, or organic compounds containing cationic or alcohol functional groups were incorporated in the form of additives. However, in his case, stability of superabsorbent polymer was lowered, or basic properties such as absorption property was deteriorated, and there were problems in terms of durability of antibacterial property and leakage of antibacterial substance.

Thus, in this invention, antibacterial substance was incorporated in superabsorbent polymer by forming a surface crosslink layer on the surface of base resin using one or more compounds selected from cationic compounds, terpene-based compounds, alcohol-based compounds, and phosphoric acid-based compounds as a surface crosslinking agent. As the result, it was confirmed that excellent bacterial growth inhibition property and deodorization property of inhibiting the growth of bacteria inducing odor existing in human skin may be continuously exhibited without deteriorating basic properties of superabsorbent polymer such as centrifuge retention capacity and absorption under pressure, and the like, and that there is no concern about leakage of antibacterial substance, thus increasing human body stability. Thus, the superabsorbent polymer can be very preferably applied for various hygienic goods such as diapers for adults, in which secondary odor particularly becomes a problem.

Specifically, the superabsorbent polymer according to one embodiment comprises base resin comprising crosslinked polymer of acrylic acid based monomers having acid groups, at least a part of the acid groups being neutralized, and an internal crosslinking agent, wherein at least a part of the base resin is surface-treated by a first surface crosslinking agent, and the first crosslinking agent is one or more selected from the group consisting of cationic compounds, terpene-based compounds, phenol-based compounds, and phosphoric acid-based compounds.

The superabsorbent polymer may be prepared by a preparation method comprising steps of: conducting crosslinking polymerization of acrylic acid-based monomers having acid groups, at least a part of the acid groups being neutralized, and an internal crosslinking agent, to form hydrogel polymer (step 1); drying, grinding and classifying the hydrogel polymer to form base resin (step 2); and crosslinking the surface of the base resin, in the presence of a first surface crosslinking agent (step 3). Thus, according to another embodiment of the invention, there is provided a method for preparing the superabsorbent polymer.

Hereinafter, the invention will be explained in detail according to steps.

First, step 1 for the preparation of the superabsorbent polymer according to one embodiment is a step of forming hydrogel polymer.

Specifically, the hydrogel polymer may be prepared by crosslinking polymerization of acrylic acid-based monomers in which at least a part of the acid groups are neutralized, and an internal crosslinking agent. For this purpose, a monomer composition comprising acrylic acid-based monomers in which at least a part of the acid groups are neutralized, a polymerization initiator, an internal crosslinking agent and a solvent, in the form of solution, may be used.

The acrylic acid-based monomer is a compound represented by the following Chemical Formula 1:

[Chemical Formula 1]     $R^1\text{-}COOM^1$

In the Chemical Formula 1,

$R^1$ is a C2-5 alkyl group comprising an unsaturated bond,
$M^1$ is a hydrogen atom, a monovalent or divalent metal, an ammonium group or an organic amine salt.

Preferably, the acrylic acid-based monomers may be one or more selected from the group consisting of acrylic acid, (meth)acrylic acid, and monovalent metal salts, divalent metal salts, ammonium salts and organic amine salts of these acids.

Wherein, the acrylic acid-based monomers have acid groups, and at least a part of the acid groups may be neutralized. Preferably, monomers partially neutralized with alkali material such as sodium hydroxide, potassium hydroxide, calcium hydroxide, ammonium hydroxide, and the like may be used. Wherein, the neutralization degree of the acrylic acid-based monomers may be 40 mol% or more, or about 45 mol% or more, and 95 mol% or less, 80 mol% or less, or 75 mol% or less. Although the range of the neutralization degree may vary according to the final properties, if the neutralization degree is too high, neutralized monomers may be precipitated, thus rendering smooth progression of polymerization difficult, and to the contrary, if the neutralization degree is too low, the absorption of the polymer may be significantly lowered, and the polymer may exhibit rubber-like property, which is difficult to handle.

The concentration of the acrylic acid-based monomers may be about 20 wt% or more, or about 40 wt% or more, and about 60 wt% or less, or about 50 wt% or less, based on the monomer composition comprising the raw materials of the superabsorbent polymer comprising acrylic acid-based monomers having acid groups of which at least a part are neutralized, a polymerization initiators and internal crosslinking agents, and solvents, and it may be appropriately adjusted considering polymerization time and reaction conditions, and the like. However, if the concentration of the monomers is too low, yield of superabsorbent polymer may decrease, thus causing a problem in terms of economical efficiency, and if the concentration is too high, the monomers may be partially precipitated or grinding efficiency during grinding of polymerized hydrogel polymer may be low, thus causing process problems, and the properties of superabsorbent polymer may be deteriorated.

And, the internal crosslinking agent is used to crosslink inside of polymer in which acrylic acid-based monomers are polymerized, and is distinguished from a surface crosslinking agent used to crosslink the surface of the polymer.

As specific examples of the internal crosslinking agent, one or more selected from the group consisting of polyethyleneglycol diacrylate, N,N'-methylenebisacrylamide, trimethylolpropane tri(meth)acrylate, ethyleneglycol di(meth)acrylate, polyethyleneglycol (meth)acrylate, propyleneglycol di(meth)acrylate, polypropyleneglycol (meth)acrylate, butanediol di(meth)acrylate, butyleneglycol di(meth)acrylate, diethyleneglycol di(meth)acrylate, hexanediol di(meth)acrylate, triethyleneglycol di(meth)acrylate, tripropyleneglycol di(meth)acrylate, tetraethyleneglycol di(meth)acrylate, dipentaerythritol pentaacrylate, glycerin tri(meth)acrylate, pentaerythritol tetraacrylate, triarylamine, ethyleneglycol diglycidyl ether, propyleneglycol, glycerin, and ethylenecarbonate may be used, but not limited thereto.

Such an internal crosslinking agent may be included in the content of 0.01 to 1 part by weight, based on 100 parts by weight of the acrylic acid-based monomers, so as to crosslink polymerized polymer. If the content of the internal crosslinking agent is less than 0.01 part by weight, improvement effect according to crosslinking may be insignificant, and if the content of the internal crosslinking agent is greater than 1 part by weight, absorption performance of superabsorbent polymer may be deteriorated. More specifically, the internal crosslinking agent may be included in the content of 0.01 parts by weight or more, or 0.05 parts by weight or more, or 0.1 parts by weight or more, and 1 part by weight or less, 0.5 parts by weight or less, or 0.3 parts by weight or less, based on 100 parts by weight of the acrylic acid-based monomers.

In the preparation method of superabsorbent polymer of the invention, the polymerization initiator used during polymerization is not specifically limited as long as it is commonly used for the preparation of superabsorbent polymer.

Specifically, as the polymerization initiator, a thermal polymerization initiator or a photopolymerization initiator according to UV irradiation may be used according to a polymerization method. However, even in the case of photopolymerization, since a certain amount of heat is generated by UV irradiation, etc., and heat is generated to some degree according to the progression of an exothermic polymerization reaction, a thermal polymerization initiator may be additionally used.

As the photopolymerization initiator, any compounds capable of forming radicals by light such as UV may be used without limitations.

As the photopolymerization initiator, one or more selected from the group consisting of benzoin ether, dialkyl acetophenone, hydroxyl alkylketone, phenyl glyoxylate, benzyl dimethyl ketal, acyl phosphine, and α-aminoketone may be used. As specific examples of the acyl phosphine, commercially available lucirin TPO, namely diphenyl(2,4,6-trimethylbenzoyl)-phosphine oxide) may be used. More various photopolymerization initiators are described in Reinhold Schwalm, "UV Coatings: Basics, Recent Developments and New Application(Elsevier 2007)", page 115, and are not limited to the above described examples.

The photopolymerization initiator may be included in the content of 0.001 to 1 part by weight, based on 100 parts by weight of the acrylic acid-based monomers. If the content of the photopolymerization initiator is less than 0.001 part by weight, polymerization speed may become slow, and if the content of the photopolymerization initiator is greater than 1 part by weight, the molecular weight of superabsorbent polymer may be small and the properties may become non-uniform. More specifically, the photopolymerization initiator may be included in an amount of 0.005 parts by weight or more, or 0.01 parts by weight or more, or 0.1 parts by weight or more, and 0.5 parts by weight or less, or 0.3 parts by weight or less, based on 100 parts by weight of the acrylic acid-based monomers.

And, in case a thermal polymerization initiator is further included as the polymerization initiators, one or more selected from the group consisting of persulfate initiators, azo initiators, hydrogen peroxide, and ascorbic acid may be used as the thermal polymerization initiator. Specific examples of the persulfate initiator may include sodium persulfate ($Na_2S_2O_8$), potassium persulfate ($K_2S_2O_8$), ammonium persulfate (($NH4)_2S_2O_8$), etc., and, specific examples of the azo initiator may include 2,2-azobis(2-amidinopropane)dihydrochloride, 2,2-azobis-(N,N-dimethylene)isobutyramidinedihydrochloride, 2-(carbamoylazo)isobutyronitril, 2,2-azobis[2-(2-imidazolin-2-yl)propane]dihydrochloride, 4,4-azobis-(4-cyanovalericacid), etc. More various thermal polymerization initiators are described in "Principle of Polymerization (Wiley, 1981)", Odian, page 203, and are not limited to the above described examples.

The thermal polymerization initiator may be included in the content of 0.001 to 1 part by weight, based on 100 parts by weight of the acrylic acid-based monomers. If the content of the thermal polymerization initiator is less than 0.001 parts by weight, additional thermal polymerization may hardly occur, and thus, the effect according to the addition of the thermal polymerization initiator may be insignificant, and if the content of the thermal polymerization initiator is greater than 1 part by weight, the molecular weight of superabsorbent polymer may be small and the properties may become non-uniform. More specifically, the thermal polymerization initiator may be included in he content of 0.005 parts by weight or more, or 0.01 parts by weight or more, or 0.1 parts by weight or more, and 0.5 parts by weight or less, or 0.3 parts by weight or less, based on 100 parts by weight of the acrylic acid-based monomers.

Besides the polymerization initiators, one or more additives such as a surfactant, a thickener, a plasticizer, a preservation stabilizer, an antioxidant, and the like may be further included as necessary.

A monomer composition comprising the above explained acrylic acid-based monomers, internal crosslinking agent, polymerization initiators, and optionally, additives, may be prepared in the form of a solution dissolved in a solvent.

Wherein, the solvent that can be used is not limited in terms of its construction as long as it can dissolve or disperse the above explained raw materials, and for example, one or more selected from water, ethanol, ethyleneglycol, diethyleneglycol, triethyleneglycol, 1,4-butanediol, propyleneglycol, ethyleneglycol monobutyl ether, propyleneglycol monomethyl ether, propyleneglycol monomethyl ether acetate, methylethylketone, acetone, methylamylketone, cyclohexanone, cyclopentanone, diethyleneglycol monomethyl ether, diethyleneglycol ethyl ether, toluene, xylene, butyrolactone, carbitol, methylcellosolve acetate and N,N-dimethylacetamide, etc. may be used alone or in combination, but not limited thereto. The solvent may be included in the remaining amount expect the above-described components, based on the total amount of the monomer composition.

Meanwhile, a method of forming hydrogel polymer by photopolymerization of the monomer composition is not specifically limited, as long as it is a commonly used polymerization method.

Specifically, the photopolymerization may be conducted at a temperature of 60°C or more, or 70°C or more. and 90°C or less, or 80°C or less, by irradiating ultraviolet rays having intensity of 5mW or more, or 10mW or more, and 30mW or less, or 20mW or less. When conducting photopolymerization under these conditions, crosslinked polymer may be formed with more excellent polymerization efficiency.

And, in case the photopolymerization is progressed, it may be progressed in a reactor equipped with a movable conveyer belt, but the above explained polymerization method is no more than one example, and the invention is not limited thereto.

And, in case photopolymerization is progressed in a reactor equipped with a movable conveyer belt as explained above, the obtained hydrogel polymer may be in the form of a sheet having the width of the belt. Wherein, the thickness of the polymer sheet may vary according to the concentration of the introduced monomer composition and the introduction speed, but, commonly, a monomer composition is preferably fed such that polymer in the form of a sheet having a thickness of about 0.5 cm to about 5 cm may be obtained. In case a monomer mixture is fed such that the thickness of sheet-shaped polymer may be too thin, production efficiency may be low, and if the thickness of the sheet-shaped polymer is greater than 5cm, due to the too thick thickness, a polymerization reaction may not uniformly occur throughout the whole thickness.

Wherein, the moisture content of hydrogel polymer obtained by such a method may be about 40 to about 80 wt%. Throughout the specification, the "moisture content" is the content of moisture occupied based on the total weight of hydrogel polymer, and it means a value obtained by subtracting the weight of polymer of a dry state from the weight of hydrogel polymer. Specifically, it is defined as a value calculated by measuring the weight loss according to moisture evaporation in the polymer during the process of drying by raising the temperature of polymer through infrared heating to dry. Wherein, the drying condition is set up such that the temperature is raised from room temperature to about 180°C and then maintained at 180°C, and the total drying time is 20 minutes including a temperature raising step of 5 minutes.

Meanwhile, after preparing the hydrogel polymer, a coarse grinding process of grinding the above prepared hydrogel polymer before subsequent drying and grinding processes may be optionally conducted.

The coarse grinding process is a process for increasing drying efficiency in the subsequent drying process, and controlling the particle size of the finally prepared superabsorbent polymer powder, wherein, grinders that can be used in the coarse grinding are not limited in terms of the constructions, but specifically, one selected from the group consisting of a vertical pulverizer, a turbo cutter, a turbo grinder, a rotary cutter mill, a cutter mill, a disc mill, a shred crusher, a crusher, a chopper, a disc cutter may be used, but are not limited thereto.

The coarse grinding process may be progressed, for example, such that particle diameter of hydrogel polymer may become about 2 to about 10 mm. Grinding to a particle diameter of less than 2 mm would not be technically easy due to the high moisture content of the hydrogel polymer, and may generate aggregation between the ground particles. Meanwhile, if grinding to a particle diameter greater than 10 mm, the effect of increasing the efficiency of the subsequent drying step may be insignificant.

Next, the step 2 is a step of drying, grinding and classifying the hydrogel polymer prepared in the step 1, to form base resin.

The drying method is not limited in terms of the construction as long as it can be commonly used as a drying process of hydrogel polymer. Specifically, the drying step may be progressed by hot wind supply, infrared ray irradiation, ultrahigh frequency wave irradiation, or UV irradiation, etc.

Specifically, the drying may be conducted at a temperature of about 150 to about 250°C. If the drying temperature is less than 150°C, drying time may too lengthen, and the properties of the finally formed superabsorbent polymer may be deteriorated, and if the drying temperature is greater than 250°C, only the surface of polymer may be dried, and thus, fines may be generated in the subsequent grinding process, and the properties of the finally formed superabsorbent polymer may be deteriorated. Thus, preferably, the drying may be progressed at a temperature of 150°C or more, or 160°C or more, and 200°C or less, or 180°C or less.

Meanwhile, the drying may be progressed for about 20 to about 90 minutes considering process efficiency, and the like, but the drying time is not limited thereto.

After progressing such a drying step, moisture content of polymer may be about 5 to about 10 wt%.

After the drying process, a grinding process is conducted.

The grinding process may be conducted such that the particle diameter of polymer powder, namely base resin may become about 150 to about 850$\mu$m. As a grinder used for grinding to such a particle diameter, specifically, a pin mill, a hammer mill, a screw mill, a roll mill, a disc mill, or a jog mill, etc. may be used, but is not limited thereto.

And, in order to manage the properties of superabsorbent polymer powder finally productized after the grinding step, a process of classifying the ground polymer powders according to particle diameter may be further conducted.

Preferably, polymer having particle diameter of about 150 to about 850$\mu$m may be classified, and using only the polymer having such particle diameter as base resin, a surface crosslinking step may be conducted to productize.

The base resin obtained by the above processes may be in the form of powder comprising crosslinked polymer that is crosslinked by acrylic acid-based monomers and internal crosslinking agent. Specifically, the base resin may be in the form of powder having particle diameter of 150 to 850$\mu$m.

Next, the step 3 is a step of heat treating the base resin prepared in the step 2 in the presence of a first surface crosslinking agent, and optionally, a second surface crosslinking agent, to surface crosslink.

The surface crosslinking is a step of increasing the crosslinking density around the surface of base resin, with regard to the crosslinking density inside particles. In general, a surface crosslinking agent is applied on the surface of base resin. Thus, this reaction mainly occurs on the surface of base resin, and it improves crosslinkability on the surface of particles without substantially affecting the inside of particles. Thus, surface cross-linked base resin has higher crosslinking degree around the surface than inside.

Such a surface crosslinking step is progressed using a surface crosslinking solution comprising the first surface crosslinking agent.

The first surface crosslinking agent used for the preparation of superabsorbent polymer according to one embodiment of the invention may be a compound having antibacterial property, and simultaneously, having functional groups that can be bonded with the carboxylic groups of base resin. Alternatively, it may be prepared by incorporating one or more functional groups that can be bonded with carboxylic groups in an antibacterial compound without functional groups that can be bonded with carboxylic group. Thereby, a surface crosslink layer having antibacterial property may be formed on the surface of the base resin.

In the first surface crosslinking agent, functional groups forming crosslink with carboxylic groups may be specifically, hydroxyl groups, epoxy groups, carbonate groups, metal salts, or amine groups, and the like, and the first surface crosslinking agent may comprise one or two or more functional groups among them.

And, it is preferable to use hydrophilic compounds as the first surface crosslinking agent used for the preparation of superabsorbent polymer according to one embodiment of the invention, so as to prevent deterioration of the original absorption properties of superabsorbent polymer. However, hydrophobic compounds may be also used as the first surface crosslinking agent, and antibacterial property may be endowed while minimizing deterioration of absorption properties through the control of the content according to the degree of hydrophobicity.

Compounds that can be used as such a first surface crosslinking agent may be one or more selected from cationic compounds, terpene-based compounds, phenol-based compounds and phosphoric acid-based compounds. These compounds exhibit excellent antibacterial property, and simultaneously, form covalent bonds with the carboxylic groups on the surface of base resin and are stably included in the surface crosslink layer, and thus, there is no concern about problem occurrence according to dissolution of antibacterial agent.

As the cationic compounds, specifically, guanidine-based compounds may be used.

The terpene-based compounds may be specifically menthol-based compounds, or citronellol-based compounds, and one or a mixture of two or more of these compounds may be used.

And, the phosphoric acid-based compounds may be specifically, phosphoric acid, phosphonic acid-based compound such as phosphonic acid or amino phosphonic acid, or phosphinic acid-based compounds such as phosphinic acid, bis(aminomethyl) phosphinic acid, or bis(hydroxymethyl) phosphinic acid.

According to one example of the invention, as the first surface crosslinking agent, cationic compounds may be used, and among the cationic compounds, guanidine-based compounds may be more preferably used.

As the examples of guanidine-based compounds that can be used, guanidine, guanidine carbonate, or guanidine hydrochloride may be mentioned, and preferably, guanidine carbonate may be used.

According to another example of the invention, as the first surface crosslinking agent, terpene-based compounds may be used, and menthol or citronellol may be more preferably used.

According to one example of the invention, as the first surface crosslinking agent, among phenol-based compounds, gallic acid, tannic acid, coumaric acid, caffeic acid, ferulic acid or protocatechuic acid may be used, and gallic acid may be more preferably used.

The first surface crosslinking agent is incorporated in the surface crosslink layer by crosslinking polymerization with base resin, and thus, is included in superabsorbent polymer. Thus, through the control of appropriate content of the first surface crosslinking agent, antibacterial and deodorization properties may be improved without deteriorating absorption properties of superabsorbent polymer.

Specifically, the superabsorbent polymer according to one embodiment of the invention may comprise the first surface crosslinking agent in the content of 0.01 to 10 parts by weight, based on 100 parts by weight of the base resin. More specifically, the first surface crosslinking agent may be included in the content of 0.01 parts by weight or more, or 0.1 parts by weight or more, or 0.5 parts by weight or more, and 10 parts by weight or less, or 5 parts by weight or less, or 3 parts by weight or less, or 2 parts by weight or less, based on 100 parts by weight of the base resin.

In case the first surface crosslinking agent is included in the above content range, excellent antibacterial and deodorization properties may be continuously and stably exhibited without concern about deterioration of the properties of superabsorbent polymer.

Meanwhile, the superabsorbent polymer according to one embodiment of the invention may further comprise a second surface crosslinking agent during surface treatment, so as to improve the original absorption properties. The second surface crosslinking agent is distinguished from the first surface crosslinking agent, in that it does no have antibacterial functional groups.

And, according to preferable example of the invention, both the first and second surface crosslinking agents are included so as to exhibit improved bacterial growth inhibition property and deodorization property without deterioration of the properties of superabsorbent polymer such as centrifuge retention capacity and absorption under pressure.

The second surface crosslinking agent is not limited in terms of is construction as long as it can react with the functional groups of the polymer. Preferably, in order to improve the properties of prepared superabsorbent polymer, as the second surface crosslinking agent, one or more selected from polyhydric alcohol-based compounds; epoxy compounds; polyamine compounds; haloepoxy compounds; condensation products of haloepoxy compounds; oxazoline compounds; mono-, di- or polyoxazolidinone compounds; cyclic urea compounds; multivalent metal salts; and alkylene carbonate-based compounds may be used.

Specifically, as the examples of the polyhydric alcohol-based compounds, one or more selected from ethyleneglycol, propyleneglycol, 1,3-propanediol, 1,4-butanediol, 1,6-hexanediol, 1,2-hexanediol, 1,3-hexanediol, 2-methyl-1,3-propanediol, 2,5-hexanediol, 2-methyl-1,3-pentanediol, 2-methyl-2,4-pentanediol, tripropyleneglycol and glycerol may be used.

And, as the epoxy compounds, ethyleneglycol diglycidyl epoxide, ethyleneglycol diglycidyl ether and glycidol, and the like may be used, and as the polyamine compounds, one or more selected from ethylenediamine, diethylenetriamine, triethylenetetramine, tetraethylenepentamine, pentaethylenehexamine, polyethyleneimine and polyamide polyamine may be used.

And, as the haloepoxy compounds, epichlorohydrin, epibromohydrin, and $\alpha$-methyl epichlorohydrin may be used. Meanwhile, as the mono-, di- or polyoxazolidinone compounds, for example, 2-oxazolidinone, and the like may be used.

As the alkylene carbonate-based compounds, C2 to 6 alkylene carbonate such as ethylene carbonate or propylene carbonate, and the like may be used. These compounds may be used alone, or two or more kinds of alkylene carbonates having different carbon numbers may be used in combination.

And, as the mulivalent metal salts, specifically, sulfate or carboxylate containing metal such as aluminum, and the like may be used, and among them, aluminum sulfate may be more preferably used.

The second surface crosslinking agent may be incorporated in the content of 0.001 to 5 parts by weight, based on 100 parts by weight of the base resin. For example, the second surface crosslinking agent may be incorporated in the content of 0.005 parts by weight or more, or 0.01 parts by weight or more, or 0.1 parts by weight or more, and 3 parts by weight or less, or 2 parts by weight or less, or 1 part by weight or less, or 0.3 parts by weight or less, based on 100 parts by weight of the base resin. By controlling the content range of the second surface crosslinking agent within the above range, superabsorbent polymer exhibiting excellent properties such as absorption performance and permeability can be prepared.

A method for mixing the first and second surface crosslinking agents with base resin is not limited in terms of its constructions. The first and second surface crosslinking agents may be put in a reactor with base resin and mixed, or the first and second surface crosslinking agents may be sprayed to base resin, or the first and second surface crosslinking agents and base resin may be continuously fed to a mixer in and mixed.

Thereby, on the base resin, a surface crosslink layer that is formed by additional crosslinking of at least a part of the base resin by the first surface crosslinking agent, or the first and second surface crosslinking agents, is formed. Preferably, on the base resin, a surface crosslink layer that is formed by uniform crosslinking of the entire surface of the base resin by the first surface crosslinking agent, or the first and second surface crosslinking agents, may be formed.

Thus, according to one embodiment of the invention, the superabsorbent polymer may be in the form of a core-shell wherein the base resin is a core, and the surface crosslink layer covers the core in the form of shell. More specifically, the superabsorbent polymer may form a core-shell structure covered with a surface crosslink layer in the form of shell formed by the first surface crosslinking agent, or a surface crosslink layer in the form of shell formed by the first and second surface crosslinking agents.

According to another embodiment of the invention, the superabsorbent polymer may be in the form of sea-island wherein the base resin is a core, and discontinuous surface crosslink structures are positioned on the core in the form of islands. More specifically, the superabsorbent polymer may form a sea-island structure wherein the base resin is a core, and on the core, surface crosslink structures formed by the first surface crosslinking agent discontinuously exist in the form of islands, or surface crosslink structures formed by the first and second surface crosslinking agents discontinuously exist in the form of islands.

In addition to the first and second surface crosslinking agents, water and alcohol may be mixed together and added in the form of a surface crosslinking solution. In case water and alcohol are added, the first and second surface crosslinking agents may be uniformly dispersed in the base resin. Wherein, the content of water and alcohol added may be about 5 to about 12 parts by weight, based on 100 parts by weight of the base resin, so as to induce uniform dispersion of the first and second surface crosslinking agents, prevent agglomeration of the bas resin, and optimize surface penetration depth of the crosslinking agent.

By heating the base resin to which the first and second surface crosslinking agents are added at a temperature of about 150 to about 220°C for about 15 to about 100 minutes, a surface crosslinking reaction may be progressed. If the crosslinking reaction temperature is less than 150 °C, a surface crosslinking reaction may not sufficiently occur, and if it is greater than 220 °C, a surface crosslinking reaction may be excessively progressed. And, if the crosslinking reaction time is less than 15 minutes, sufficient crosslinking reaction may not be progressed, and if it is greater than 100 minutes, due to excessive surface crosslinking reaction, crosslinking density of particle surface may become excessively high, and thus, properties may be deteriorated. More specifically, the surface crosslinking reaction may be progressed by heating at a temperature of 150°C or more, or 160°C or more, and 220°C or less, or 200°C or less, for 20 minutes or more, or 40 minutes or more, and 70 minutes or less, or 60 minutes or less.

A temperature rise means for the additional crosslinking reaction is not specifically limited. A heating medium may be supplied, or a heat source may be directly supplied to heat. Wherein, the kinds of the heating medium that can be used may include temperature-increased fluid such as steam, hot air, hot oil, etc., but are not limited thereto, and the temperature of the heating medium may be appropriately selected considering the means of the heating medium, temperature rise speed and a temperature to be increased. Meanwhile, the heat source directly supplied may include electric heating, gas heating, etc., but is not limited thereto.

Through the above preparation process, superabsorbent polymer can be prepared and provided.

The prepared superabsorbent polymer comprises: base resin comprising crosslinked polymer of acrylic acid-based monomers comprising acid groups, at least a part the acid groups being neutralized, and an internal crosslinking agent; and a surface crosslink layer that is formed on the base resin, by additional crosslinking of the base resin by the first surface crosslinking agent, or the first and second surface crosslinking agents. As such, by including antibacterial agent-derived units on the surface crosslink layer of base resin, improved bacterial growth inhibition property and deodorization property may be continuously and safely exhibited without deterioration of the properties of superabsorbent polymer such as centrifuge retention capacity and absorption under pressure, and the like. And, since the antibacterial substance is included while being physically or chemically strongly fixed or bonded to the base resin, unlike the case of simply mixing an antibacterial agent with superabsorbent polymer, non-uniform application, delamination and separation during transportation may not occur, and antibacterial components may be uniformly included, thus stably exhibiting excellent bacterial growth inhibition property and deodorization property for a long time.

The superabsorbent polymer wherein at least a part of the base resin is surface treated by the first surface crosslinking agent may have centrifuge retention capacity(CRC) measured according to EDANA method WSP 241.3, in the range of about 28 g/g or more, or about 29 g/g or more, or about 30 g/g or more and about 60 g/g or less, or about 55 g/g or less, or about 50 g/g or less, or about 40 g/g or less, or about 38 g/g or less, or about 35 g/g or less.

And, the superabsorbent polymer wherein at least a part of the base resin is surface treated by the first surface crosslinking agent may have absorption under pressure(AUP) of 0.7 psi, measured according to EDANA method WSP 242.3, in the range of about 8 g/g or more, or 10 g/g or more, or about 20 g/g or more, or about 23 g/g or more, or about 25 g/g or more and about 37 g/g or less, or about 35 g/g or less, or about 32 g/g or less.

The superabsorbent polymer wherein at least a part of base resin is surface treated by the first surface crosslinking agent and second surface crosslinking agent may have centrifuge retention capacity(CRC) measured according to EDANA method WSP 241.3, in the range of about 28 g/g or more, or about 29 g/g or more, or about 30 g/g or more and about 40 g/g or less, or about 38 g/g or less, or about 35 g/g or less.

And, the superabsorbent polymer wherein at least a part of base resin is surface treated by the first surface crosslinking agent and second surface crosslinking agent may have absorption under pressure(AUP) of 0.7 psi, measured according to EDANA method WSP 242.3, in the range of about 20 g/g or more, or about 23 g/g or more, or about 25 g/g or more and about 37 g/g or less, or about 35 g/g or less, or about 32 g/g or less.

Thus, the superabsorbent polymer may be very preferably included and used in various hygienic goods, such as diapers for children, diapers for adults, or sanitary pads, and the like, and particularly, it may be very preferably applied for diapers for adults, in which secondary odor caused by bacterial growth particularly becomes a problem.

Such hygienic goods may have common constructions of hygienic goods, except comprising the superabsorbent polymer of one embodiment in the absorber.

And, such superabsorbent polymer may be used for various articles, for example, absorbent goods, water-holding material for soil, water stop material for civil engineering and architecture, sheet for raising seedling, freshness preservatives, fomentation materials, electric insulators, oral or dental articles, articles for cosmetics or skin, besides hygienic goods.

The invention will be explained in more detail in the following Examples. However, these examples are presented only as the illustrations of the invention, and the scope of the invention is not limited thereby.

**<Example>**

**Preparation of superabsorbent polymer**

**Example 1**

In a 3L glass container equipped with a stirrer, a nitrogen doser and a thermometer, 518g of acrylic acid, 1.2g of polyethyleneglycol (400) diacrylate and 0.04g of diphenyl(2,4,6-trimethylbenzoyl)-phosphine oxide were added and dissolved, and then, 822.2g of 24.5% sodium hydroxide solution was added, and while continuously dosing nitrogen, an aqueous solution of water-soluble unsaturated monomers was prepared. The aqueous solution of water-soluble unsaturated monomers was cooled to 40°C, and 15.54g of 4% sodium persulfate aqueous solution was added, and then, the aqueous solution was added to a container of width 250mm, length 250mm, and height 30mm, made of stainless, and irradiated by ultraviolet rays in a 80°C UV chamber for 60 seconds (irradiation dose: $10mV/cm^2$)) and aged for 2 minutes to obtain hydrogel polymer. The obtained hydrogel polymer was ground to 2mm * 2mm, and then, the obtained gel type resin was spread to a thickness of 30mm on a stainless wire gauze having a hole size of 600 $\mu$m, and dried in a 185°C hot air oven for 32 minutes. The dried polymer thus obtained was ground using a grinder, and classified with ASTM standard sieve, thus obtaining base resin having particle size of 150 to 850$\mu$m.

To 100 parts by weight of the base resin, a surface crosslinking solution comprising 4.4 parts by weight of water, 0.3 parts by weight of ethylene carbonate, 0.075 parts by weight of polycarboxylic acid salt surfactant, 0.3 parts by weight of aluminum sulfate, and 0.5 parts by weight of guanidine carbonate was sprayed and mixed, the mixture was put in a container consisting of a stirrer and double jacket, and surface crosslinking reaction was progressed at 180°C for 70 minutes. And then, the surface-treated powder was classified with ASTM standard sieve to obtain superabsorbent polymer powder having particle size of 150 to 850$\mu$m.

**Example 2**

Base resin was prepared by the same method as Example 1, and then, using 100 parts by weight of the base resin, and a surface crosslinking solution comprising 4.4 parts by weight of water, 0.3 parts by weight of ethylene carbonate, 0.075 parts by weight of polycarboxylic acid salt surfactant, 0.3 parts by weight of aluminum sulfate and, 0.5 parts by weight of menthol, surface crosslinking was progressed by the same method as Example 1. Subsequent process was progressed by the same method as Example 1 to obtain superabsorbent polymer powder.

**Example 3**

Base resin was prepared by the same method as Example 1, and then, using 100 parts by weight of the base resin, and a surface crosslinking solution comprising 4.4 parts by weight of water, 0.3 parts by weight of ethylene carbonate, 0.075 parts by weight of polycarboxylic acid salt surfactant, 0.3 parts by weight of aluminum sulfate and, 0.5 parts by weight of gallic acid, surface crosslinking was progressed by the same method as Example 1. Subsequent process was progressed by the same method as Example 1 to obtain superabsorbent polymer powder.

**Example 4**

Base resin was prepared by the same method as Example 1, and then, using 100 parts by weight of the base resin, and a surface crosslinking solution comprising 4.4 parts by weight of water, 0.3 parts by weight of ethylene carbonate, 0.075 parts by weight of polycarboxylic acid salt surfactant, 0.3 parts by weight of aluminum sulfate and, 0.5 parts by weight of citronellol, surface crosslinking was progressed by the same method as Example 1. Subsequent process was progressed by the same method as Example 1 to obtain superabsorbent polymer powder.

### Example 5

Base resin was prepared by the same method as Example 1, and then, using 100 parts by weight of the base resin, and a surface crosslinking solution comprising 4.4 parts by weight of water, 0.3 parts by weight of ethylene carbonate, 0.075 parts by weight of polycarboxylic acid salt surfactant, 0.3 parts by weight of aluminum sulfate and, 2 parts by weight of phosphoric acid, surface crosslinking was progressed by the same method as Example 1. Subsequent process was progressed by the same method as Example 1 to obtain superabsorbent polymer powder.

### Example 6

Base resin was prepared by the same method as Example 1, and then, using 100 parts by weight of the base resin, and a surface crosslinking solution comprising 4.4 parts by weight of water, 0.075 parts by weight of polycarboxylic acid salt surfactant, 0.3 parts by weight of aluminum sulfate and, 2 parts by weight of phosphoric acid, surface crosslinking was progressed by the same method as Example 1. Subsequent process was progressed by the same method as Example 1 to obtain superabsorbent polymer powder.

### Comparative Example 1

Base resin was prepared by the same method as Example 1, and then, using 100 parts by weight of the base resin, and a surface crosslinking solution comprising 4.4 parts by weight of water, 0.3 parts by weight of ethylene carbonate, 0.075 parts by weight of polycarboxylic acid salt surfactant, and 0.3 parts by weight of aluminum sulfate, surface crosslinking was progressed by the same method as Example 1.

And then, the surface-treated powder was classified with ASTM standard sieve to obtain superabsorbent polymer powder having particle size of 150 to 850 $\mu$m.

### Comparative Example 2

100 parts by weight of the superabsorbent polymer powder prepared by the method of Comparative Example 1 was dry mixed with 0.5 parts by weight of guanidine carbonate, without a solvent at a temperature of 25°C, using a Vortex mixer (Vortex-Genie 2 mixer, Scientific Industries).

### Comparative Example 3

100 parts by weight of the superabsorbent polymer powder prepared by the method of Comparative Example 1 was dry mixed with 0.5 parts by weight of menthol by the same method as Comparative Example 2.

### Comparative Example 4

100 parts by weight of the superabsorbent polymer powder prepared by the method of Comparative Example 1 was dry mixed with 0.5 parts by weight of gallic acid by the same method as Comparative Example 2.

### Comparative Example 5

100 parts by weight of the superabsorbent polymer powder prepared by the method of Comparative Example 1 was dry mixed with 0.5 parts by weight of citronellol by the same method as Comparative Example 2.

### Comparative Example 6

100 parts by weight of the superabsorbent polymer powder prepared by the method of Comparative Example 1 was dry mixed with 2 parts by weight of phosphoric acid by the same method as Comparative Example 2.

### <Experimental Example>

For each superabsorbent polymer prepared in Examples and Comparative Examples, the properties were evaluated as follows.

Unless otherwise described, the following property evaluations were progressed at constant temperature constant humidity(23±1 °C, relative humidity 50±10%), and physiological saline or saline solution means an aqueous solution of 0.9 wt% sodium chloride.

(1) Centrifuge Retention Capacity(CRC)

Centrifuge retention capacity(CRC) by absorption rate under no load of each polymer was measured according to EDANA WSP 241.3.

Specifically, $W_0$ (g, about 0.2g) of the superabsorbent polymers were uniformly put in an envelope made of non-woven fabric, and the envelope was sealed, and then, soaked in a saline solution(0.9 wt%) at room temperature. After 30 minutes, the envelope was drained at 250G for 3 minutes using a centrifuge, and then, the weight $W_2(g)$ of the envelope was measured. And, after the same operation was conducted using an empty envelope without a sample, the weight Wi(g) at that time was measured. Using the obtained weights, CRC (g/g) was calculated according to the following Formula.

[Mathematical Formula 1]

$$CRC \ (g/g) = \{[W_2(g) - W_1(g)]/W_0(g)\} - 1$$

(2) Absorption Under Pressure(AUP)

Absorption under pressure of 0.7 psi of each polymer was measured according to EDANA method WSP 242.3.

Specifically, a 400 mesh wire netting made of stainless was installed on the bottom of a plastic cylinder with an inner diameter of 60 mm. Under the conditions of room temperature and relative humidity of 50%, $W_0$(g, 0.90 g) of superabsorbent polymer were uniformly scattered on the wire netting, and a piston that can uniformly apply a load of 0.7 psi was put on the superabsorbent polymer. Wherein, as the piston, a piston having an outer diameter slightly smaller than 60 mm was used such that there was no gap with the inner wall of the cylinder, and the movement upward and downward was not hindered. At this time, the weight $W_3(g)$ of the apparatus was measured.

Subsequently, on the inner side of a petri dish having a diameter of 150 mm, a glass filter having a diameter of 90 mm and a thickness of 5 mm was put, and a 0.9 wt% sodium chloride aqueous solution (saline solution) was poured on the petri dish. Wherein, the saline solution was poured until the water level of the saline solution became the same level to the upper side of the glass filter. And, one filter paper with a diameter of 90 mm was put thereon. On the filer paper, the above prepared apparatus was mounted, and the liquid was absorbed for 1 hour under load. After 1 hour, the measurement apparatus was raised, and the weight W4(g) was measured.

Using the measured weights, absorption under pressure(g/g) was calculated according to the following Formula.

[Mathematical Formula 2]

$$AUP(g/g) = [W_4(g) - W_3(g)]/W_0(g)$$

(3) Bacterial growth inhibition performance test 1

50ml of artificial urine inoculated with 3000 CFU/ml of Proteus Mirabilis (ATCC 29906) was incubated in a 37°C incubator for 16 hours. The artificial urine immediately after the inoculation of bacteria and the artificial urine 16 hours after the inoculation were used as controls, and they were sufficiently washed with 150ml of saline, and cultured on a Nutrient broth agar(BD DIFCO.) plate, and CFU(Colony Forming Unit; CFU/ml) was measured to calculate the properties of controls.

More specifically, 2g of each superabsorbent polymer of Examples 1 to 4 and Comparative Examples 1 to 5 was added to 50ml of the artificial urine inoculated with 3000 CFU/ml of Proteus Mirabilis (ATCC 29906), and shaken for 1 minute to uniformly mix. It was incubated in a 37°C incubator for 16 hours. The artificial urine after incubated for 16 hours was sufficiently washed with 150ml of saline and cultured on a Nutrient broth agar plate, and CFU(Colony Forming Unit; CFU/ml) was measured.

Each measurement result was used to calculate Proteus Mirabilis (ATCC 29906) growth rate represented by the following Formula 1, and based thereon, bacterial growth inhibition property of each Example and Comparative Example was evaluated:
A strain used to measure bacterial growth rate, Proteus Mirabilis, secretes urease, and the urease decomposes urea in urine, thus generating ammonia causing odor. Thus, antibacterial and deodorization effects of superabsorbent polymer can be properly evaluated by Proteus Mirabilis growth rate. Meanwhile, in case the growth of Proteus Mirabilis is excessively inhibited so as to achieve deodorization effect, other bacteria beneficial to the human body may be also killed, and thus, problems such as skin rash, and the like may be caused. Thus, appropriate level of growth inhibition is required.

[Formula 1]

Bacterial growth rate(%) = [CFU(16h)]/ [CFU(0h)] * 100

In the Formula 1, CFU(0h) denotes the number of bacteria(Proteus Mirabilis; ATCC29906) initially inoculated to superabsorbent polymer per unit volume of artificial urine (3000 CFU/ml), and CFU(16h) denotes the number of grown bacteria per unit volume of artificial urine, when the superabsorbent polymer was maintained at 37°C for 16 hours (CFU/ml).

The property values of Examples and Comparative Examples are summarized in the following Table 1.

[Table 1]

| | Example 1 | Example 2 | Example 3 | Example 4 | Comparative Example 1 | Comparative Example 2 | Comparative Example 3 | Comparative Example 4 | Comparative Example 5 |
|---|---|---|---|---|---|---|---|---|---|
| Centrifuge retention capacity ( CRC) (g/g) | 36.2 | 36.4 | 36.2 | 36.1 | 37.2 | 37.2 | 37.5 | 37.4 | 37.2 |
| Absorptio n under pressure of 0.7psi(AUP) (g/g) | 21.6 | 22.0 | 21.5 | 21.1 | 21.6 | 21.1 | 20.0 | 21.4 | 21.2 |
| CFU(16h) (CFU/ml) | $3.94*10^5$ | $8.02*10^6$ | $1.03*10^7$ | $3.42*10^6$ | $1.36*10^7$ | $3.45*10^6$ | $1.04*10^7$ | $1.31*10^7$ | $1.04*10^7$ |
| Bacterial growth rate (%) | 2.9 | 59.0 | 75.4 | 25.1 | 100 | 25.4 | 76.5 | 96.2 | 76.5 |

Referring to Table 1, it is confirmed that in the case of Examples 1 to 3 wherein surface crosslinking is conducted using the first surface crosslinking agent of the invention, excellent bacterial growth inhibition property may be exhibited without deterioration of absorption properties.

In the case of Comparative Examples 2 to 4 wherein the same compound was dry mixed with surface crosslinked superabsorbent polymer, although bacterial growth rate was lower than Comparative Example 1 wherein an antibacterial compound was not added, bacterial growth rate was about 20% higher than each of Examples 1 to 3. Thus, it can be confirmed that in case a specific antibacterial compound is used as a surface crosslinking agent to progress surface crosslinking, compared to the case of simply mixing it with superabsorbent polymer, excellent bacterial growth inhibition property and deodorization property may be stably exhibited for a long time.

(4) Bacterial growth inhibition performance test 2

50ml of artificial urine inoculated with 3000 CFU/ml of Proteus Mirabilis (CCUG 4637) was incubated in a 35°C incubator for 12 hours. The artificial urine immediately after the inoculation of bacteria and the artificial urine 12 hours after the inoculation were used as controls, they were sufficiently washed with 150ml of saline and cultured on a Nutrient broth agar(BD DIFCO.) plate, and CFU(Colony Forming Unit; CFU/ml) was measured to calculate the properties of controls.

More specifically, 2g of each superabsorbent polymer of Examples 5 to 6 and Comparative Examples 1, 6 and 7 was added to 50ml of the artificial urine inoculated with 3000 CFU/ml of Proteus Mirabilis (CCUG 4637), and shaken for 1 minute to uniformly mix. It was incubated in a 35°C incubator for 12 hours. The artificial urine after incubated for 12 hours was sufficiently washed with 150ml of saline and cultured on a Nutrient broth agar plate, and CFU(Colony Forming Unit; CFU/ml) was measured.

Each measurement result was used to calculate Proteus Mirabilis (CCUG 4637) growth rate represented by the following Formula 2, and based thereon, bacterial growth inhibition property of each Example and Comparative Example was evaluated:

[Formula 2]

$$\text{Bacterial growth rate}(\%) = [\text{CFU(12h)}]/ [\text{CFU(0h)}] * 100$$

In the Formula 2, CFU(0h) denotes the number of bacteria(Proteus Mirabilis; CCUG 4637) initially inoculated to superabsorbent polymer per unit volume of artificial urine (3000 CFU/ml), and CFU(12h) denotes the number of grown bacteria per unit volume of artificial urine, when the superabsorbent polymer was maintained at 35°C for 12 hours (CFU/ml).

[Table 2]

|  | Example 5 | Example 6 | Comparative Example1 | Comparative Example 6 |
|---|---|---|---|---|
| Centrifuge retention capacity (CRC) (g/g) | 36.8 | 53.2 | 37.2 | 37.5 |
| Absorption under pressure of 0.7psi (AUP) (g/g) | 21.7 | 8.1 | 21.6 | 21.6 |
| CFU(12h) (CFU/ml) | $4.53*10^4$ | $5.12*10^4$ | $1.86*10^7$ | $8.52*10^7$ |
| Bacterial growth rate (%) | 0.24 | 0.28 | 100 | 45.8 |

Referring to Table 2, it is confirmed that when using a phosphoric acid-based compound as the first surface crosslinking agent, excellent bacterial growth inhibition property may be also exhibited. Meanwhile, in the case of Example 6 wherein surface crosslinking was progressed using only the first surface crosslinking agent without using the second surface crosslinking agent, although antibacterial performance comparable to Example 5 was exhibited, there was difference in the properties of superabsorbent polymer

The microorganism existing in the artificial urine uniformly exists inside and outside the superabsorbent polymer. However, in case the same compound was dry mixed with surface crosslinked superabsorbent polymer, the antibacterial compound is non-uniformly distributed around the superabsorbent polymer. To the contrary, in case the antibacterial compound is uniformly distributed on the surface of superabsorbent polymer through surface crosslinking, contact between the microorganism and antibacterial compound may be increased to exhibit more effective antibacterial performance.

Comparing the growth rates of Table 1 and Table 2, it was confirmed that compared to Comparative Examples wherein the antibacterial compound was simply mixed with superabsorbent polymer without surface treatment, in Examples wherein the antibacterial compound was bonded to superabsorbent polymer through surface crosslinking, microorganism growth inhibition effect was exhibited for a long incubation time of 12 hours or 16 hours.

**Claims**

**1.** Superabsorbent polymer comprising

base resin comprising crosslinked polymer of acrylic acid-based monomers having acid groups, at least a part of the acid groups being neutralized, and an internal crosslinking agent,
wherein at least a part of the base resin is surface-treated by a first surface crosslinking agent, and
the first surface crosslinking agent is one or more selected from the group consisting of cationic compounds, terpene-based compounds, phenol-based compounds, and phosphoric acid-based compounds.

**2.** The superabsorbent polymer according to claim 1, wherein the cationic compound is a guanidine-based compound.

3. The superabsorbent polymer according to claim 2, wherein the guanidine-based compound is guanidine carbonate, or guanidine hydrochloride.

4. The superabsorbent polymer according to claim 1, wherein the terpene-based compound is menthol, or citronellol.

5. The superabsorbent polymer according to claim 1, wherein the phenol-based compound is gallic acid, tannic acid, coumaric acid, caffeic acid, ferulic acid or protocatechuic acid.

6. The superabsorbent polymer according to claim 1, wherein the phosphoric acid-based compound is phosphoric acid, phosphonic acid, amino phosphonic acid, phosphinic acid, bis(aminomethyl) phosphinic acid, or bis(hydroxymethyl) phosphinic acid.

7. The superabsorbent polymer according to claim 1, wherein at least a part of the base resin is surface-treated by a first surface crosslinking agent and a second surface crosslinking agent.

8. The superabsorbent polymer according to claim 7, wherein the second surface crosslinking agent comprises one or more selected from polyhydric alcohol-based compounds; epoxy compounds; polyamine compounds; haloepoxy compounds; condensation products of haloepoxy compounds; oxazoline compounds; mono-, di- or polyoxazolidinone compounds; cyclic urea compounds; multivalent metal salts; and alkylene carbonate-based compounds.

9. The superabsorbent polymer according to claim 1, wherein the first surface crosslinking agent is included in an amount of 0.01 to 10 parts by weight, based on 100 parts by weight of the base resin.

10. The superabsorbent polymer according to claim 1, wherein centrifuge retention capacity(CRC) measured according to EDANA method WSP 241.3 is 28 g/g to 60 g/g.

11. The superabsorbent polymer according to claim 1, wherein absorption under pressure(AUP) of 0.7 psi, measured according to EDANA method WSP 242.3 is 8 g/g to 37 g/g.

12. The superabsorbent polymer according to claim 1, wherein the superabsorbent polymer inhibits growth of Proteus mirabilis bacteria.

13. The superabsorbent polymer according to claim 1, wherein the superabsorbent polymer has a core-shell structure in which the base resin is a core, and a surface crosslink layer covers the core in the form of a shell.

14. The superabsorbent polymer according to claim 7, wherein centrifuge retention capacity(CRC) measured according to EDANA method WSP 241.3 is 28 g/g to 40 g/g.

15. The superabsorbent polymer according to claim 7, wherein absorption under pressure(AUP) of 0.7 psi, measured according to EDANA method WSP 242.3, is 20 g/g to 37 g/g.

16. The superabsorbent polymer according to claim 7, wherein the superabsorbent polymer inhibits growth of Proteus mirabilis bacteria.

17. The superabsorbent polymer according to claim 7, wherein the superabsorbent polymer has a core-shell structure in which the base resin is a core, and a surface crosslink layer covers the core in the form of a shell.

18. A method for preparing superabsorbent polymer, comprising steps of:

   conducting crosslinking polymerization of acrylic acid-based monomers having acid groups, at least a part of the acid groups being neutralized, and an internal crosslinking agent, to form hydrogel polymer;
   drying, grinding and classifying the hydrogel polymer to form base resin; and
   crosslinking the surface of the base resin, in the presence of a first surface crosslinking agent,
   wherein the first surface crosslinking agent is one or more selected from cationic compounds, terpene-based compounds, phenol-based compounds, and phosphoric acid-based compounds.

19. The method for preparing superabsorbent polymer according to claim 18, wherein the step of crosslinking the surface of the base resin is conducted at 150 to 220°C.

**20.** The method for preparing superabsorbent polymer according to claim 18, wherein the step of crosslinking the surface of the base resin further comprises a second surface crosslinking agent.

**21.** The method for preparing superabsorbent polymer according to claim 20, wherein the second surface crosslinking agent is one or more selected from polyhydric alcohol-based compounds; epoxy compounds; polyamine compounds; haloepoxy compounds; condensation products of haloepoxy compounds; oxazoline compounds; mono-, di- or polyoxazolidinone compounds; cyclic urea compounds; multivalent metal salts; and alkylene carbonate-based compounds.

**22.** An article comprising superabsorbent polymer according to any one of claims 1 to 17.

**23.** The article according to claim 22, wherein the article is one or more selected from absorbent goods, hygienic goods, water-holding material for soil, water stop material for civil engineering and architecture, sheet for raising seedling, freshness preservatives, fomentation materials, electric insulators, oral or dental articles, articles for cosmetics or skin.

<div align="center">**INTERNATIONAL SEARCH REPORT**</div>

| International application No. |
| --- |
| **PCT/KR2021/004673** |

| A. | CLASSIFICATION OF SUBJECT MATTER |
| --- | --- |

**C08F 20/06**(2006.01)i; **C08K 5/00**(2006.01)i; **C08K 5/13**(2006.01)i; **C08K 5/109**(2006.01)i; **C08J 3/24**(2006.01)i; **A61L 15/60**(2006.01)i

According to International Patent Classification (IPC) or to both national classification and IPC

| B. | FIELDS SEARCHED |
| --- | --- |

Minimum documentation searched (classification system followed by classification symbols)

C08F 20/06(2006.01); C08F 220/06(2006.01); C08F 251/02(2006.01); C08J 3/075(2006.01); C08J 3/24(2006.01); C08K 3/24(2006.01); C08L 33/02(2006.01); D01F 1/10(2006.01); D01F 8/18(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 고흡수성 수지(super absorbent polymer), 구아니딘 카보네이트(guanidine carbonate), 테르펜(terpene), 페놀(phenol), 인산(phosphonic acid), 가교제(crosslinker)

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| X | KR 10-2016-0010516 A (EVONIK DEGUSSA GMBH) 27 January 2016 (2016-01-27)<br>See claims 1 and 19-24; and paragraphs [0080], [0123]-[0127] and [0138]. | 1,4-23 |
| Y | | 2,3 |
| Y | CN 106832347 A (NATIONAL DONG HWA UNIVERSITY) 13 June 2017 (2017-06-13)<br>See claims 1 and 7. | 2,3 |
| A | KR 10-2020-0036606 A (LG CHEM, LTD.) 07 April 2020 (2020-04-07)<br>See claims 1, 6 and 7. | 1-23 |
| A | CN 110067042 A (DONGHUA UNIVERSITY) 30 July 2019 (2019-07-30)<br>See claims 1 and 2. | 1-23 |
| A | JP 2019-116636 A (EVONIK CORP.) 18 July 2019 (2019-07-18)<br>See entire document. | 1-23 |

☐ Further documents are listed in the continuation of Box C.     ☑ See patent family annex.

| * | Special categories of cited documents: | | |
| --- | --- | --- | --- |
| "A" | document defining the general state of the art which is not considered to be of particular relevance | "T" | later document published after the international filing date or priority date and not in conflict with the application but cited to understand the principle or theory underlying the invention |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
| --- | --- |
| **28 July 2021** | **29 July 2021** |

| Name and mailing address of the ISA/KR | Authorized officer |
| --- | --- |
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2019)

## INTERNATIONAL SEARCH REPORT
### Information on patent family members

International application No.

**PCT/KR2021/004673**

| Patent document cited in search report | | | Publication date (day/month/year) | Patent family member(s) | | | Publication date (day/month/year) |
|---|---|---|---|---|---|---|---|
| KR | 10-2016-0010516 | A | 27 January 2016 | BR | 112015028304 | A2 | 25 July 2017 |
| | | | | BR | 112015028304 | B1 | 18 May 2021 |
| | | | | CN | 105377921 | A | 02 March 2016 |
| | | | | CN | 105377921 | B | 28 September 2018 |
| | | | | EP | 2997057 | A1 | 23 March 2016 |
| | | | | EP | 2997057 | B1 | 01 January 2020 |
| | | | | JP | 2016-524634 | A | 18 August 2016 |
| | | | | JP | 6314211 | B2 | 18 April 2018 |
| | | | | TW | 201443111 | A | 16 November 2014 |
| | | | | TW | I495669 | B | 11 August 2015 |
| | | | | US | 1001692 | B2 | 11 May 2021 |
| | | | | US | 2016-0096944 | A1 | 07 April 2016 |
| | | | | WO | 2014-183987 | A1 | 20 November 2014 |
| CN | 106832347 | A | 13 June 2017 | CN | 106832347 | B | 08 March 2019 |
| KR | 10-2020-0036606 | A | 07 April 2020 | BR | 112020023271 | A2 | 23 February 2021 |
| | | | | CN | 112004865 | A | 27 November 2020 |
| | | | | EP | 3763771 | A1 | 13 January 2021 |
| | | | | WO | 2020-067705 | A1 | 02 April 2020 |
| CN | 110067042 | A | 30 July 2019 | None | | | |
| JP | 2019-116636 | A | 18 July 2019 | None | | | |

Form PCT/ISA/210 (patent family annex) (July 2019)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 1020200044874

- KR 1020210047956

**Non-patent literature cited in the description**

- **REINHOLD SCHWALM.** UV Coatings: Basics, Recent Developments and New Application. Elsevier, 2007, 115

- **ODIAN.** Principle of Polymerization. Wiley, 1981, 203